# EUROPEAN PATENT APPLICATION

(11) **EP 3 530 727 A1**
(43) Date of publication of application: **28.08.2019**
(21) Application number: 17898114.8
(22) Date of filing: 27.02.2017
(51) Int. Cl.: C12M 3/00, C12N 15/09

(54) **SOMATIC CELL PRODUCTION SYSTEM**

(71) Applicant: Tanabe, Koji, Palo Alto, CA 94303 (US); I Peace, Inc., Palo Alto, CA 94303 (US)
(72) Inventor: TANABE, Koji, Palo Alto, California 94304 (US); SUTO, Kenta, Palo Alto, CA 94303 (US); HIRAIDE, Ryoji, Kyoto-shi Kyoto 606-8414 (JP)
(74) Representative: MacLean, Martin Robert
(86) International application number: PCT/JP2017/007564
(87) International publication number: WO 2018/154788

(57) **Abstract**

A somatic cell production system comprising a preintroduction cell solution-feeding channel 20 through which a preintroduction cell-containing solution passes, a factor introducing device 30 that is connected to the preintroduction cell solution-feeding channel 20 and introduces a somatic cell inducing factor into preintroduction cells to prepare inducing factor-introduced cells, and a cell preparation device 40 in which the inducing factor-introduced cells are cultured to prepare somatic cells.

## Description

### FIELD

The present invention relates to somatic cell induction technology, and particularly to a somatic cell production system.

### BACKGROUND

Embryonic stem cells (ES cells) are stem cells established from early embryos of human or mice. ES cells are pluripotent, being capable of differentiating into all cells in the body. At the current time, human ES cells are able to be used in cell transplantation therapy for numerous diseases including Parkinson's disease, juvenile onset diabetes and leukemia. However, certain barriers exist against transplantation of ES cells. In particular, transplantation of ES cells can provoke immunorejection similar to the rejection encountered after unsuccessful organ transplantation. Moreover, there are many ethical considerations as well as critical and dissenting opinions against the use of ES cell lines that have been established by destruction of human embryos.

It was against this background that Professor Shinya Yamanaka of Kyoto University successfully established a line of induced pluripotent stem cells (iPS cells) by transferring four genes: Oct3/4, Klf4, c-Myc and Sox2, into somatic cells. For this, Professor Yamanaka received the Nobel Prize in Physiology or Medicine in 2012 (see PTL 1, for example). iPS cells are ideal pluripotent cells which are free of issues of rejection or ethical problems. Therefore, iPS cells are considered promising for use in cell transplantation therapy. In recent years, techniques have also been established allowing creation of specific cells from other cells by transfer of specific genes into the cells. Such techniques are expected to be applicable for transplant medicine and drug screening, similar to iPS cells.

Numerous methods for altering iPS cells to somatic cells exist in the prior art. For utilization of iPS cells in transplantation therapy, however, it is important to establish highly efficient differentiation-inducing methods for iPS cells. Specifically, it is necessary to establish techniques to be used for inducing differentiation of iPS cells to somatic cells, improving the differentiation-inducing efficiency and precision and ensuring that the functionality of the created somatic cells is able to withstand transplantation therapy.

Methods for inducing differentiation of iPS cells or embryonic stem cells (ES cells) to somatic cells have included methods that imitate the process of development, by combining hormones or growth factors that are the determinants of the properties of the cells, as well as low molecular compounds, and varying their quantity ratios or concentrations with time. However, it is difficult to completely emulate the development process *in vitro,* and efficiency is also poor. Moreover, inducing differentiation of human somatic cells requires a much longer differentiation-inducing period than for mice, with 3 months or longer, for example, being necessary to prepare mature nerves. Another problem is that differentiation-inducing efficiency differs widely depending on the type of ES/iPS cells, while the properties of induced somatic cells are non-homogeneous.

Specifically, cells whose differentiation has been induced from human ES/iPS cells by methods utilizing hormones or chemical substances have been confirmed to be fetal-stage somatic cells in the initial stages. It is extremely difficult to induce differentiation of mature human somatic cells, and their culturing requires long periods of several months. However, for innovative drug development and transplant medicine for fully developed individuals, it is very important to prepare somatic cells that match the maturation level of the individual.

For neurons, which include cells of a variety of different subtypes, it is not possible to induce differentiation of neuronal subtypes in a uniform manner from ES/iPS cells by methods utilizing hormones or chemical substances. Therefore, innovative drug screening specific for designated neuronal subtypes is not possible. This lowers the efficiency for innovative drug screening. For transplant medicine as well, it is not possible to concentrate and transplant only specific diseased cells.

For this reason, methods have been proposed wherein genes for the properties of specific somatic cells are directly transferred into ES/iPS cells using viruses, to create the desired somatic cells. Methods using viruses allow specific creation of mature neurons in very short time periods compared to methods using hormones or chemical substances, such as 2 weeks, for example. Moreover, creating neurons by specific gene transfer allows excitatory nerves alone, for example, to be obtained in a homogeneous manner. Therefore, specific innovative drug screening for specific neuronal subtypes becomes possible, potentially making it possible to concentrate and transplant only cells specific to a disease, for transplant medicine.

When iPS cells have been differentiated to somatic cells, there is a risk of undifferentiated iPS cells remaining among the differentiated somatic cells. Methods have therefore also been established for differentiating somatic cells into different somatic cells without requiring iPS cells. Specifically, methods have been developed for differentiating fibroblasts into myocardial cells or neurons. Such methods are known as direct reprogramming, and because they do not involve pluripotent stem cells such as iPS cells there is no risk of undifferentiated pluripotent cells remaining at the time of transplantation.

### [CITATION LIST]

### [PATENT LITERATURE]

[PTL 1]: Japanese Patent Publication No. 4183742

### SUMMARY

### [TECHNICAL PROBLEM]

Somatic cells are established by introducing inducing factors such as genes into cells which are then subjected to amplifying culturing, and cryopreserved if necessary. However, the following problems are involved in the preparation and industrialization of somatic cells for clinical use (for example, GLP or GMP grade).

### 1) Cost

Somatic cells for clinical use must be prepared and stored in a cleanroom kept in a state of very high cleanliness. The cost for maintaining the required level of cleanliness, however, is extremely high. The preparation of somatic cells for clinical use is therefore very costly, and this has been a great hindrance against industrialization.

### 2) Quality

The series of operations from establishment of somatic cells to their storage are complex, and many of them must be carried out by hand. Moreover, the preparation of somatic cells often depends on a personal level of skill. Therefore, the quality of somatic cells for clinical use varies depending on the preparer and on the particular experimental batch.

### 3) Time

In order to prevent cross-contamination with cells other than those of a particular donor in the cleanroom, somatic cells for clinical use from only a single individual are prepared in the same cleanroom over a prescribed period of time. In addition, long time periods are necessary to establish somatic cells for clinical use and evaluate their quality. However, since somatic cells for clinical use are only prepared once for a single individual in the cleanroom, a very long period of time becomes necessary to prepare somatic cells for clinical use for many different individuals.

### 4) Personnel

As mentioned above, currently the preparation of somatic cells for clinical use is for the most part carried out by hand. Nevertheless, few technicians have the skills necessary for them to prepare somatic cells for clinical use.

To counter this problem, it is an object of the present invention to provide a somatic cell production system that allows production of somatic cells. Incidentally, the somatic cells are not limited to somatic cells for clinical use.

### [SOLUTION TO PROBLEM]

According to one aspect of the invention there is provided a somatic cell production system comprising a preintroduction cell solution-feeding channel through which a preintroduction cell-containing solution passes, a factor introducing device that is connected to the preintroduction cell solution-feeding channel and introduces a somatic cell inducing factor into preintroduction cells to prepare inducing factor-introduced cells, and a cell preparation device in which the inducing factor-introduced cells are cultured to prepare somatic cells.

The somatic cell production system may further comprise an enclosure that houses the preintroduction cell solution-feeding channel, factor introducing device and cell preparation device.

In this somatic cell production system, somatic cells created by introduction of a somatic cell inducing factor may have the pluripotent stem cells removed. The somatic cells created by introduction of a somatic cell inducing factor may also include differentiated cells. The somatic cells created by introduction of a somatic cell inducing factor may also include somatic stem cells. Somatic stem cells are also known as adult stem cells or tissue stem cells. The somatic cells created by introduction of a somatic cell inducing factor may also include nervous system cells. The somatic cells created by introduction of a somatic cell inducing factor may also include fibroblasts. The somatic cells created by introduction of a somatic cell inducing factor may also include myocardial cells, keratinocytes or retinal cells.

In this somatic cell production system, the preintroduction cells may include pluripotent stem cells. The pluripotent stem cells may also include ES cells and iPS cells. The preintroduction cells may still further include somatic stem cells. The preintroduction cells may yet still further include differentiated somatic cells. The preintroduction cells may yet still further include blood cells. The preintroduction cells may yet still further include fibroblasts.

In this somatic cell production system, the cell preparation device may comprise a somatic cell culturing apparatus wherein inducing factor-introduced cells created by a factor introducing device are cultured and an amplifying culturing apparatus wherein somatic cells established by the somatic cell culturing apparatus are subjected to amplifying culturing, the somatic cell culturing apparatus optionally comprising a first culture medium supply device that supplies culture medium to the inducing factor-introduced cells, and the amplifying culturing apparatus optionally comprising a second culture medium supply device that supplies culture medium to the somatic cells.

The somatic cell culturing apparatus in the somatic cell production system may further comprise a drug supply device that feeds a solution containing a drug that kills cells in which a drug resistance factor has not been introduced.

The factor introducing device in the somatic cell production system may also comprise a factor introducing device connected to the preintroduction cell solution-feeding channel, a factor storing device that stores the somatic cell inducing factor, a factor solution-feeding channel for streaming of the somatic cell inducing factor from the factor storing device to the preintroduction cell solution-feeding channel or factor introducing device, and a pump for streaming of the liquid in the factor solution-feeding channel.

In the somatic cell production system, the somatic cell inducing factor may be DNA, RNA or protein.

In the somatic cell production system, the somatic cell inducing factor may be introduced into the preintroduction cells by RNA lipofection at the factor introducing device.

In the somatic cell production system, the somatic cell inducing factor may be incorporated into a vector. The vector may be Sendai virus vector.

The somatic cell production system may further comprise a packaging device that packages the somatic cells created by the cell preparation device, and the enclosure may house the packaging device.

The somatic cell production system described above may still further comprise a solution exchanger comprising a tubular member and a liquid permeable filter disposed inside the tubular member, the solution exchanger being provided with, in the tubular member, a somatic cell introduction hole for introduction of a solution including somatic cells created by the cell preparation device, onto the liquid permeable filter, an exchange solution introduction hole for introduction of exchange solution onto the liquid permeable filter, a somatic cell outflow hole for outflow of the exchange solution including the somatic cells onto the liquid permeable filter, and a waste liquid outflow hole through which the solution that has permeated the liquid permeable filter flows out.

The somatic cell production system may further comprise a waste liquid solution-feeding channel connected to the waste liquid outflow hole of the solution exchanger, permitting the solution containing the somatic cells to flow through the waste liquid solution-feeding channel when the solution is discarded, or not permitting the solution to flow through the waste liquid solution-feeding channel when the somatic cells are being dispersed in the exchange solution.

The exchange solution in the somatic cell production system may be a cryopreservation liquid.

The somatic cell production system may further comprise a separating device that separates preintroduction cells from blood, with the preintroduction cell-containing solution separated by the separating device optionally passing through the preintroduction cell solution-feeding channel.

### [ADVANTAGEOUS EFFECTS OF INVENTION]

According to the invention it is possible to provide a somatic cell production system that allows production of somatic cells.

### BRIEF DESCRIPTION OF DRAWINGS

Fig. 1 is a schematic view of a somatic cell production system according to an embodiment of the invention.
Fig. 2 is a schematic view of a somatic cell production system according to an embodiment of the invention.
Fig. 3 is a schematic cross-sectional view of an example of an introduced cell solution-feeding channel in a somatic cell production system according to an embodiment of the invention.
Fig. 4 is a schematic cross-sectional view of an example of an introduced cell solution-feeding channel in a somatic cell production system according to an embodiment of the invention.
Fig. 5 is a schematic view of a culturing bag to be used in a somatic cell production system according to an embodiment of the invention.
Fig. 6 is a schematic view of a solution exchanger according to an embodiment of the invention.
Fig. 7 is a schematic view of a somatic cell production system according to an embodiment of the invention.
Fig. 8 is a set of photographs of cells for Example 1.
Fig. 9 is a set of photographs of cells for Example 1.
Fig. 10 is a graph showing transfection efficiency and survival rate percentages for Example 1.
Fig. 11 is a set of photographs of cells for Example 2.
Fig. 12 is a graph showing the proportion of TUJ-1 positive cells for Example 2.
Fig. 13 is a graph showing the proportion of TUJ-1 positive cells for Example 2.
Fig. 14 is a schematic diagram illustrating transfection for Example 3.
Fig. 15 is a set of photographs of cells for Example 3.

### DESCRIPTION OF EMBODIMENTS

An embodiment of the invention will now be explained. In the accompanying drawings, identical or similar parts will be indicated by identical or similar reference numerals. However, the drawings are only schematic representations. The specific dimensions, therefore, should be judged in light of the following explanation. Furthermore, this naturally includes parts that have different dimensional relationships and proportions between drawings.

The present disclosure includes an invention that has been provisionally filed in the U.S. (62/356,199), and has already been issued a foreign application permit.

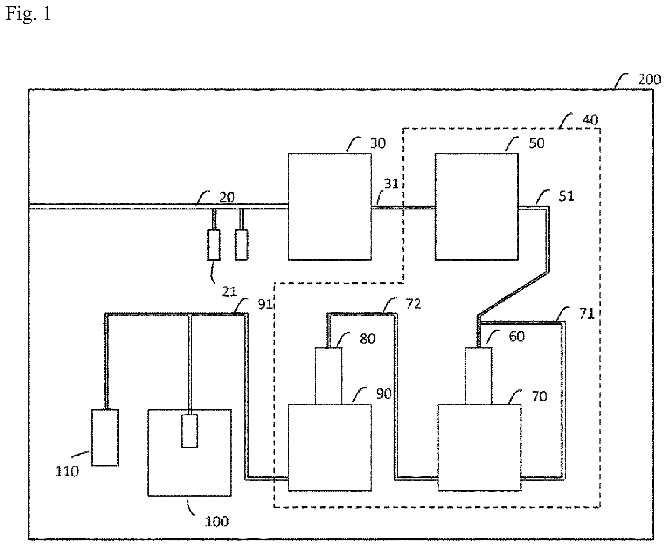

The somatic cell production system according to an embodiment of the invention, as shown in Fig. 1, comprises a preintroduction cell solution-feeding channel 20 through which a preintroduction cell-containing solution passes, a factor introducing device 30 that is connected to the preintroduction cell solution-feeding channel 20 and introduces a somatic cell inducing factor into preintroduction cells to prepare inducing factor-introduced cells, a cell preparation device 40 in which the inducing factor-introduced cells are cultured to prepare somatic cells, and an enclosure 200 that houses the preintroduction cell solution-feeding channel 20, factor introducing device 30 and cell preparation device 40.

The preintroduction cells are pluripotent stem cells, for example. ES cells and iPS cells may be used as pluripotent stem cells. Alternatively, the preintroduction cells may be differentiated cells, for example. Somatic cells differentiated from somatic stem cells, blood cells and fibroblasts may be used as differentiated cells. Somatic stem cells are also known as adult stem cells or tissue stem cells.

The somatic cells created by introduction of a somatic cell inducing factor exclude pluripotent stem cells. The somatic cells created by introduction of a somatic cell inducing factor are differentiated cells. Differentiated cells include somatic stem cells, nervous system cells, fibroblasts, myocardial cells, hepatocytes, retinal cells, cornea cells, blood cells, keratinocytes and chondrocytes. Nervous system cells may be neurons, neural stem cells or neural precursor cells. Neurons may be inhibitory neurons, excitatory neurons or dopamine-producing neurons. Alternatively, nervous system cells may be motor nerve cells, oligodendrocyte progenitor cells or oligodendrocytes. Nervous system cells may be MAP2-positive or β-III Tubulin-positive.

The somatic cell production system still further comprises an air purifier that purifies the gas in the enclosure 200, a temperature regulating device that regulates the temperature of the gas in the enclosure 200, and a carbon dioxide concentration control device that controls the concentration of carbon dioxide (CO₂) in the gas in the enclosure 200. The air purifier may also comprise a cleanliness sensor that monitors the cleanliness of the gas in the enclosure 200. The air purifier purifies the air in the enclosure 200 using a HEPA (High Efficiency Particulate Air) filter or ULPA (Ultra Low Penetration Air) filter, for example. The air purifier purifies the air in the enclosure 200 to a cleanliness conforming to ISO standard 14644-1, class ISO1 to ISO6, for example. The temperature regulating device may also comprise a temperature sensor that monitors the temperature of the gas in the enclosure 200. The CO₂ concentration control device may also comprise a CO₂ concentration sensor that monitors the CO₂ concentration of the gas in the enclosure 200.

A door or the like is provided in the enclosure 200, the interior being completely sealed when the door is closed, allowing constant cleanliness, temperature and CO₂ concentration to be maintained for the air in the interior. The enclosure 200 is preferably transparent so as to allow observation of the state of the interior devices from the outside. The enclosure 200 may also be a glove box integrated with gloves, such as rubber gloves.

An inlet communicating with the preintroduction cell solution-feeding channel 20 may also be provided in the enclosure 200. A door or the like may also be provided at the opening. Alternatively, the inlet may be closeable with a removable sealing material. The preintroduction cells are accommodated in the preintroduction cell solution-feeding channel 20 through the inlet. Alternatively, a preintroduction cell-storing vessel that stores the preintroduction cells and communicates with the preintroduction cell solution-feeding channel 20, may be disposed inside the enclosure 200.

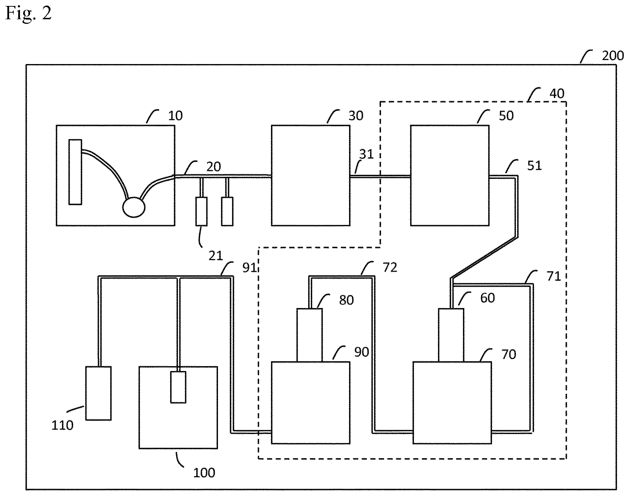

As yet another alternative, the somatic cell production system may further comprise a separating device 10 that separates the preintroduction cells from blood, disposed inside the enclosure 200 as shown in Fig. 2. In this case the preintroduction cell solution-feeding channel 20 is connected to the separating device 10. Preintroduction cell-containing solution that has been separated by the separating device 10 passes through the preintroduction cell solution-feeding channel 20.

The separating device 10 in the enclosure 200 receives vials containing human blood, for example. The separating device 10 comprises an anticoagulant tank that stores anticoagulants such as ethylenediaminetetraacetic acid (EDTA), heparin and biologically standardized blood storage Solution A (ACD Solution A, product of Terumo Corp.), for example. The separating device 10 employs a pump or the like to add an anticoagulant to human blood from the anticoagulant tank.

In addition, the separating device 10 comprises a separating reagent tank that stores a mononuclear cell separating reagent such as Ficoll-Paque PREMIUM^{R} (product of GE Healthcare, Japan). The separating device 10 employs a pump or the like to inject 5 mL of mononuclear cell separating reagent from the separating reagent tank into each of two 15 mL tubes, for example. Resin bags may be used instead of tubes.

The separating device 10 also comprises a buffering solution tank that stores a buffering solution such as phosphate-buffered saline (PBS). The separating device 10 employs a pump to add 5 mL of buffering solution from the buffering solution tank to 5 mL of human blood, for example, to dilute it. The separating device 10 additionally employs a pump or the like to add 5 mL of the diluted human blood to each of the mononuclear cell separating reagents in the tubes.

The separating device 10 further comprises a temperature-adjustable centrifuge. The centrifuge may be set to 18°C, for example. The separating device 10 employs a moving apparatus or the like to place the tubes in which the mononuclear cell separating reagent and human blood have been placed, into holders of the centrifuge. The centrifuge performs centrifugation of the solutions in the tubes for 30 minutes at 400 × g, for example. Resin bags may be centrifuged instead of tubes.

After centrifugation, the separating device 10 collects the intermediate layers that have become turbid and white by the mononuclear cells in the solutions in the tubes, using a pump or the like. The separating device 10 employs a pump or the like to deliver the recovered mononuclear cell suspensions to the preintroduction cell solution-feeding channel 20. Alternatively, the separating device 10 also adds 12 mL of PBS, for example, to 2 mL of the recovered mononuclear cell solutions, and places the tubes in holders of the centrifuge. The centrifuge performs centrifugation of the solutions in the tubes for 10 minutes at 200 × g, for example.

After centrifugation, the separating device 10 employs a pump or the like to remove the supernatants of the solutions in the tubes by suction, and adds 3 mL of mononuclear cell culture medium such as X-VIVO 10^{R} (Lonza, Japan) to the mononuclear cell solutions in the tubes to prepare suspensions. The blood cells may be cultured in a feeder-free manner using a basal membrane matrix such as Matrigel (Corning), CELLstart^{R} (ThermoFisher) or Laminin 511 (Nippi). The separating device 10 employs a pump or the like to deliver the mononuclear cell suspension as preintroduction cells to the preintroduction cell solution-feeding channel 20. The separating device 10 may also employ a dialysis membrane to separate the mononuclear cells from the blood. When previously prepared preintroduction cells are used, the separating device 10 may be omitted.

The separating device 10 may also separate cells suitable for induction by a method other than centrifugal separation. For example, if the cells to be separated are T cells, cells that are CD3-, CD4- or CD8-positive may be separated by panning. If the cells to be separated are vascular endothelial precursor cells, then cells that are CD34-positive may be separated by panning. If the cells to be separated are B cells, cells that are CD10-, CD19- or CD20-positive may be separated by panning. The separation may also be carried out by a magnetic-activated cell sorting (MACS) method or flow cytometry, without limitation to panning.

The inner wall of the preintroduction cell solution-feeding channel 20 may be coated with poly-HEMA (poly 2-hydroxyethyl methacrylate) to render it non-cell-adherent, so that the preintroduction cells do not adhere. Alternatively, a material resistant to adhesion of the preintroduction cells may be used as the material for the preintroduction cell solution-feeding channel 20. By using a material with good thermal diffusivity and CO₂ permeability, for example, as the material of the preintroduction cell solution-feeding channel 20, the conditions in the preintroduction cell solution-feeding channel 20 can be rendered equivalent to the controlled temperature and CO₂ concentration in the enclosure 200. In addition, a back-flow valve may be provided in the preintroduction cell solution-feeding channel 20 from the viewpoint of preventing contamination.

The inducing factor solution-feeding mechanism 21 in the enclosure 200 comprises, for example, an inducing factor-introducing reagent tank that stores an inducing factor-introducing reagent solution. The inducing factor solution-feeding mechanism 21 employs a micropump or the like to deliver the inducing factor-introducing reagent solution to the preintroduction cell solution-feeding channel 20 or factor introducing device 30 in the enclosure 200, in such a manner that the suspension of preintroduction cells is suspended in the inducing factor-introducing reagent solution.

The inducing factor-introducing reagent solution, such as a gene transfer reagent solution, includes a set comprising somatic cell inducing factor RNA, RNA transfection solution and RNA transfection culture medium, for example. RNA transfection also includes RNA lipofection. The somatic cell inducing factor RNA set includes, for example, 100 ng each of ASCL1 mRNA, Myt1L mRNA and neurogenin 2 (Ngn2) mRNA. Ngn2 (neurogenin 2) is a switch protein necessary for nervous system cell differentiation.

The somatic cell inducing factor RNA may include mRNA corresponding to a drug resistance gene. A "drug" is, for example, an antibiotic such as puromycin, neomycin, blasticidin, G418, hygromycin or Zeocin. The cells into which mRNA corresponding to a drug resistance gene has been introduced will exhibit drug resistance. Somatic cell inducing factor RNA includes Ngn2-T2A-Puro mRNA (Trilink), for example. Cells transfected with Ngn2-T2A-Puro mRNA (Trilink) produce neurogenin 2 (Ngn2) and exhibit puromycin resistance.

The mRNA may be capped with Anti-Reverse Cap Analog (ARCA) and polyadenylated, and optionally substituted with 5-methylcytidine and pseudouridine. The ability of antibody to recognize mRNA is reduced by 5-methylcytidine and pseudouridine.

The RNA transfection solution includes small interfering RNA (siRNA) or a lipofection reagent, for example. An siRNA lipofection reagent or mRNA lipofection reagent may be used as RNA lipofection reagents. More specifically, the RNA lipofection reagent used may be Lipofectamine^{R} RNAiMAX (Thermo Fisher Scientific), Lipofectamine^{R} MessengerMAX (Thermo Fisher Scientific), Lipofectamin^{R} 2000, Lipofectamin^{R} 3000, NeonTransfection System (Thermo Fisher scientific), Stemfect RNA transfection reagent (Stemfect), NextFect^{R} RNA Transfection Reagent (BiooSientific), Amaxa^{R} Human T cellNucleofector^{R} kit (Lonza, VAPA-1002), Amaxa^{R} Human CD34 cell Nucleofector^{R} kit (Lonza, VAPA-1003), ReproRNA^{R} transfection reagent STEMCELL Technologies) or mRNA-in^{R} (Thermo fisher scientific).

As an example, the factor introducing device 30 may introduce the somatic cell inducing factor into the cells and then suspend the cells in culture solution. The factor introducing device 30 may also carry out transfection of the somatic cell inducing factor several times. After a prescribed time period such as 24 hours, for example, after introducing the somatic cell inducing factor into the cells, the medium may be exchanged and the somatic cell inducing factor again transfected into the cells. The transfection of the somatic cell inducing factor into the cells, and the cell culturing for the prescribed time period, may be repeated several times, such as 2 to 4 times.

During lipofection of the somatic cell inducing factor RNA, when using a 12-well plate, for example, the number of cells per well is from 1 × 10⁴ to 1 × 10⁸, from 5 × 10⁴ to 1 × 10⁶ or from 1 × 10⁵ to 5 × 10⁵. The base area per well is 4 cm². The amount of somatic cell inducing factor RNA during lipofection of the somatic cell inducing factor RNA is from 200 ng to 5000 ng, from 400 ng to 2000 ng or from 500 ng to 1000 ng, each time. The amount of lipofection reagent during lipofection of the somatic cell inducing factor RNA is from 0.1 µL to 100 µL, from 1 µL to 50 µL or from 1.5 µL to 10 µL.

The culture medium used during lipofection of the somatic cell inducing factor RNA is low serum medium such as Opti-MEM^{R} (Gibco). The medium used during, and before and after, lipofection of the somatic cell inducing factor RNA may also include B18R protein. B18R protein reduces congenital antiviral reaction of the cells. B18R protein is sometimes used to inhibit cell death due to immunoreaction during insertion of RNA into cells. However, if the cells are to be differentiated into somatic cells in a short period of time, the medium does not need to include B18R protein, or it may contain B18R protein in a low concentration of 0.01% to 1%.

Animal cells differentiate into somatic cells within 10 days, 9 days, 8 days or 7 days from lipofection of the somatic cell inducing factor RNA. When the somatic cells to be created are nervous system cells, differentiation into nervous system cells can be confirmed by whether or not they are positive for β-III Tubulin, MAP2 or PsA-NCAM. B-III Tubulin, MAP2, PsA-NCAM and vGlu are neuron-identifying markers, being constituent proteins of microtubules in neuronal processes.

Alternatively, the inducing factor-introducing reagent solution such as a gene transfer reagent solution may include a Sendai virus vector solution. RNA derived from Sendai virus is not integrated into host DNA, but it allows a gene of interest to be introduced into the host. A Sendai virus vector set includes ASCL1 mRNA, Myt1L mRNA and Ngn2 mRNA, for example, with a MOI (multiplicity of infection) of 0.01 to 1000, 0.1 to 1 or 1 to 10. The inducing factor/Sendai virus vector may also include mRNA corresponding to a drug resistance gene. The inducing factor RNA included in the Sendai virus vector may include Ngn2-T2A-Puro mRNA (Trilink), for example. Introduction of the Sendai virus into the cells may be carried out once.

The factor introducing device 30 feeds the solution containing the cells into which the inducing factor has been introduced (inducing factor-introduced cells) into the introduced cell solution-feeding channel 31 using a pump or the like.

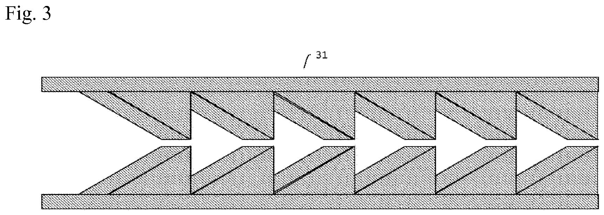

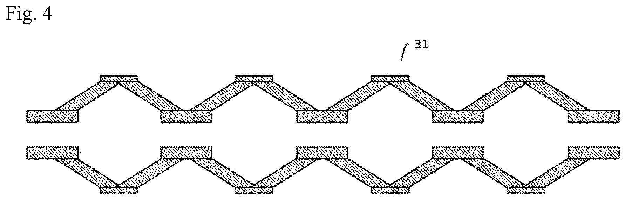

The inner wall of the introduced cell solution-feeding channel 31 in the enclosure 200 may be coated with poly-HEMA to render it non-adhesive, so that the inducing factor-introduced cells do not adhere. Alternatively, a material resistant to adhesion of the inducing factor-introduced cells may be used as the material for the introduced cell solution-feeding channel 31. Also, by using a material with good thermal diffusivity and CO₂ permeability, for example, as the material of the introduced cell solution-feeding channel 31, the conditions in the introduced cell solution-feeding channel 31 will be equivalent to the controlled temperature and CO₂ concentration in the enclosure 200. In addition, a back-flow valve may be provided in the introduced cell solution-feeding channel 31 from the viewpoint of preventing contamination. Also, as shown in Fig. 3, one or a plurality of folds may be formed in the interior of the introduced cell solution-feeding channel 31 to intermittently vary the inner diameter. As another alternative, the inner diameter of the introduced cell solution-feeding channel 31 may be intermittently varied, as shown in Fig. 4.

As shown in Fig. 1 and Fig. 2, the cell preparation device 40 connected to the introduced cell solution-feeding channel 31 comprises a somatic cell culturing apparatus 50 in which the inducing factor-introduced cells prepared at the factor introducing device 30 are cultured, a first dissociating mechanism 60 that dissociates the cell mass (cell colonies) comprising somatic cells established at the somatic cell culturing apparatus 50 into a plurality of cell masses, an amplifying culturing apparatus 70 that carries out amplifying culturing of the somatic cells, a second dissociating mechanism 80 that dissociates the cell mass comprising somatic cells that have been cultured by amplifying culturing at the amplifying culturing apparatus 70 into a plurality of cell masses, and a somatic cell transport mechanism 90 that delivers the somatic cells in order to a packaging device 100. When no cell mass is formed, however, or when the cell mass does not need to be dissociated, the first dissociating mechanism 60 and second dissociating mechanism 80 may be omitted.

The somatic cell culturing apparatus 50 may also comprise a culturing vessel including a well plate, bag and tube inside it. The somatic cell culturing apparatus 50 may further comprise a pipetting machine. The somatic cell culturing apparatus 50 receives the solution containing the somatic cell inducing factor-introduced cells from the introduced cell solution-feeding channel 31, and allocates the solution into the culturing vessel by the pipetting machine.

When the cells are to be differentiated into nervous system cells, the inducing factor-introduced cells are placed in the culture vessel of the somatic cell culturing apparatus 50, and then from the 1st to 7th day, for example, N3 medium (DMEM/F12, 25 µg/mL insulin, 50 µg/mL human transferrin, 30 nmol/L sodium selenite, 20 nmol/L progesterone, 100 nmol/L putrescine) as nerve differentiation medium is added to the culture vessel. ROCK inhibitor (Selleck) may also be added to the medium at a concentration of 10 µmol/L for several days.

The inducing factor-introduced cells are allocated to the culture vessel in the somatic cell culturing apparatus 50, and then on the 9th day, for example, the medium is exchanged, with medium exchange being carried out thereafter until the target cells such as nervous system cells are observed. The "medium exchange" includes partial exchange of the culture medium, as well as replenishment.

In the somatic cell culturing apparatus 50, drug selection may be carried out, whereby cells into which the drug resistance factor has not been introduced are killed. When the somatic cell inducing factor RNA contains mRNA corresponding to a drug resistance gene, a solution containing the drug is supplied to the culture vessel and the inducing factor-introduced cells exhibiting drug resistance selectively survive. For example, when the somatic cell inducing factor RNA includes mRNA corresponding to a puromycin resistance gene, the lipofected cells may be exposed to puromycin to kill the cells other than those in which the somatic cell inducing factor RNA has been introduced, and select out the cells in which the somatic cell inducing factor RNA has been introduced. The drug may also be present in the medium. The drug concentration may be 2 mg/L, for example.

In the somatic cell culturing apparatus 50, the inducing factor-introduced cells are cultured for a prescribed period of time using medium containing a drug that kills cells in which the drug resistance factor has not been introduced, after which the inducing factor-introduced cells are cultured in medium lacking the drug.

When the target somatic cells are formed, the somatic cell culturing apparatus 50 collects the somatic cells with a pipetting machine. In addition, the somatic cell culturing apparatus 50 places a vessel containing the collected somatic cells in an incubator, and reacts the somatic cells with the trypsin-substituting recombinant enzyme for 10 minutes at 37°C, 5% CO₂. When the cell masses are to be physically disrupted, there is no need for a trypsin-substituting recombinant enzyme. For example, the somatic cell culturing apparatus 50 disrupts the cell masses of the somatic cells by pipetting with a pipetting machine. Alternatively, the somatic cell culturing apparatus 50 may disrupt the cell masses by passing the cell masses through a pipe provided with a filter, or a pipe that intermittently varies the inner diameter, similar to the introduced cell solution-feeding channel 31 shown in Fig. 3 or Fig. 4.

For example, when nerves are to be induced, the somatic cell culturing apparatus 50 subsequently adds nerve differentiation medium as described above to a solution containing the disrupted cell masses of the somatic cells.

Culturing in the somatic cell culturing apparatus 50 may be carried out in a bag instead of a well plate. The bag may also be CO₂-permeable. The culturing may be by adhesion culture or suspension culture. In the case of suspension culture, agitation culture may be carried out. Culturing in the somatic cell culturing apparatus 50 may also be hanging drop culturing.

The somatic cell culturing apparatus 50 may also comprise a first culture medium supply device that supplies medium containing a culture solution, inside the culture vessel including the well plate, bag and tube. The first culture medium supply device collects the culture solution in the culture vessel, and it may use a filter or dialysis membrane to filter the culture solution, to allow reuse of the purified culture solution. During this time, growth factors or the like may be added to the culture solution that is to be reused. The somatic cell culturing apparatus 50 may also comprise, in the culture vessel, a drug supply device that feeds a solution containing a drug that kills cells in which a drug resistance factor has not been introduced. The somatic cell culturing apparatus 50 may further comprise a temperature regulating device that regulates the temperature of the medium, a pH control device that controls the pH of the medium, and a humidity regulating device that regulates the humidity surrounding the medium.

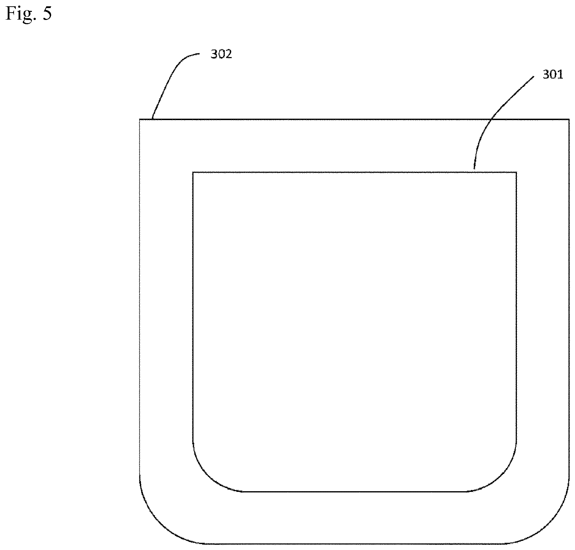

In the somatic cell culturing apparatus 50, the cells may be placed in a culture solution-permeable bag 301 such as a dialysis membrane, as shown in Fig. 5, the culture solution-permeable bag 301 may be placed in a culture solution-impermeable bag 302, and the culture solution may be placed in the bags 301, 302. The bag 302 may be CO₂-permeable or CO₂-impermeable. The somatic cell culturing apparatus 50 may have multiple bags 302 prepared containing fresh culture solution, and the bag 302 in which the cell-containing bag 301 is placed may be replaced by an outer bag 302 containing fresh culture solution, at prescribed intervals of time.

A first somatic cell solution feeding channel 51 is connected to the somatic cell culturing apparatus 50 shown in Fig. 1 and Fig. 2. The somatic cell culturing apparatus 50 feeds the solution containing the somatic cells to the first somatic cell solution feeding channel 51 using a pump or the like. The first somatic cell solution feeding channel 51 may have an inner diameter that allows passage of only induced cells of less than a prescribed size, and it may be connected to a branched fluid channel that removes non-induced cells of a prescribed size or larger.

The inner wall of the first somatic cell solution feeding channel 51 may be coated with poly-HEMA to render it non-cell-adherent, so that the somatic cells do not adhere. Alternatively, a material resistant to somatic cell adhesion may be used as the material for the first somatic cell solution feeding channel 51. Also, by using a material with good thermal diffusivity and CO₂ permeability as the material of the first somatic cell solution feeding channel 51, the conditions in the first somatic cell solution feeding channel 51 will be equivalent to the controlled temperature and CO₂ concentration in the enclosure 200. In addition, a back-flow valve may be provided in the first somatic cell solution feeding channel 51 from the viewpoint of preventing contamination.

The first somatic cell solution feeding channel 51 is connected to the first dissociating mechanism 60. The first dissociating mechanism 60 comprises a mesh, for example. The cell masses in the solution are dissociated into a plurality of cell masses of the sizes of the holes of the mesh, when they pass through the mesh by water pressure. If the mesh hole sizes are uniform, for example, the sizes of the plurality of cell masses after being dissociated will be approximately uniform. Alternatively, the first dissociating mechanism 60 may comprise a nozzle. For example, if the interior of an approximately conical nozzle is micromachined in a step-wise manner, a cell mass in the solution will be dissociated into a plurality of cell masses when it passes through the nozzle.

The amplifying culturing apparatus 70 is connected to the first dissociating mechanism 60. The solution including cell masses of the somatic cells that have been dissociated at the first dissociating mechanism 60 is fed to the amplifying culturing apparatus 70. When cell masses do not form, the first dissociating mechanism 60 may be omitted. In this case, the first somatic cell solution feeding channel 51 is connected to the amplifying culturing apparatus 70.

The amplifying culturing apparatus 70 can house a well plate in its interior, for example. The amplifying culturing apparatus 70 also comprises a pipetting machine. The amplifying culturing apparatus 70 receives the solution including the somatic cells from the first dissociating mechanism 60 or first somatic cell solution feeding channel 51, and the solution is allocated into the wells with a pipetting machine. After allocating the somatic cells into the wells, the amplifying culturing apparatus 70 cultures the somatic cells for about 8 days, for example, at 37°C, 5% CO₂. The amplifying culturing apparatus 70 also carries out appropriate exchange of the culture medium.

When cell masses are formed, the amplifying culturing apparatus 70 then adds a trypsin-substituting recombinant enzyme such as TrypLE Select^{R} (Life Technologies Corp.) to the cell masses. In addition, the amplifying culturing apparatus 70 raises the temperature of the vessel containing the cell masses, and reacts the cell masses with the trypsin-substituting recombinant enzyme for 1 minute at 37°C, 5% CO₂. When the cell masses are to be physically disrupted, there is no need for a trypsin-substituting recombinant enzyme. For example, the amplifying culturing apparatus 70 disrupts the cell masses by pipetting with a pipetting machine. Alternatively, the amplifying culturing apparatus 70 may disrupt the cell masses by passing the cell masses through a pipe provided with a filter, or a pipe that intermittently varies the inner diameter, similar to the introduced cell solution-feeding channel 31 shown in Fig. 3 or Fig. 4. The amplifying culturing apparatus 70 shown in Fig. 1 and Fig. 2 then adds culture medium such as maintenance culture medium to the solution containing the cell masses. Furthermore, when the amplifying culturing apparatus 70 carries out adhesion culture, the cell masses are scraped from the vessel with an automatic cell scraper or the like, and the cell mass-containing solution is fed to the first dissociating mechanism 60 through an amplifying culturing solution-feeding channel 71.

Culturing in the amplifying culturing apparatus 70 may be carried out in a bag or tube instead of a well plate. The bag or tube may be CO₂-permeable. In addition, the culturing may be by adhesion culture, or by suspension culture, or by hanging drop culture. In the case of suspension culture, agitation culture may be carried out.

The amplifying culturing apparatus 70 may also comprise a second culture medium supply device that supplies culture solution to the culture vessel including the well plate, bag and tube. The second culture medium supply device collects the culture solution in the culture vessel, and it may use a filter or dialysis membrane to filter the culture solution, to allow reuse of the purified culture solution. During this time, growth factors or the like may be added to the culture solution that is to be reused. The amplifying culturing apparatus 70 may also comprise a temperature regulating device that regulates the temperature of the culture medium, and a humidity regulating device that regulates the humidity in the vicinity of the culture medium.

In the amplifying culturing apparatus 70, the cells may be placed in a culture solution-permeable bag 301 such as a dialysis membrane, as shown in Fig. 5, the culture solution-permeable bag 301 may be placed in a culture solution-impermeable bag 302, and the culture solution may be placed in the bags 301, 302. The bag 302 may also be CO₂-permeable. The amplifying culturing apparatus 70 may have multiple bags 302 prepared containing fresh culture solution, and the bag 302 in which the cell-containing bag 301 is placed may be replaced by an outer bag 302 containing fresh culture solution, at prescribed intervals of time.

The somatic cell production system shown in Fig. 1 and Fig. 2 may further comprise a photographing device that photographically records culturing in the somatic cell culturing apparatus 50 and amplifying culturing apparatus 70. If a colorless culture medium is used for the culture medium in the somatic cell culturing apparatus 50 and amplifying culturing apparatus 70, it will be possible to minimize diffuse reflection and autologous fluorescence that may be produced when using a colored culture medium. In order to confirm the pH of the culture medium, however, a pH indicator such as phenol red may be included. Moreover, since induced cells and non-induced cells have differences in cellular shape and size, the somatic cell production system may further comprise an induced state monitoring device that calculates the proportion of induced cells by photographing the cells in the somatic cell culturing apparatus 50 and amplifying culturing apparatus 70. Alternatively, the induced state monitoring device may determine the proportion of induced cells by antibody immunostaining or RNA extraction. In addition, the somatic cell production system may comprise a non-induced cell removing device that removes cells that have not been induced, by magnetic-activated cell sorting, flow cytometry or the like.

The cell masses that have been dissociated by the first dissociating mechanism 60 shown in Fig. 1 and Fig. 2 are again cultured in the amplifying culturing apparatus 70. Dissociation of the cell masses at the first dissociating mechanism 60 and culturing of the somatic cells in the amplifying culturing apparatus 70 are repeated until the necessary cell volume is obtained. When cell masses do not form, the first dissociating mechanism 60 may be omitted, as mentioned above.

A second somatic cell solution feeding channel 72 is connected to the amplifying culturing apparatus 70. The amplifying culturing apparatus 70 feeds the solution containing the amplifying cultured somatic cells to the second somatic cell solution feeding channel 72 using a pump or the like. Detachment is not necessary, however, in the case of suspension culture. The second somatic cell solution feeding channel 72 may have an inner diameter that allows passage of only induced somatic cells of less than a prescribed size, and it may be connected to a branched fluid channel that removes non-induced cells of a prescribed size or larger.

The inner wall of the second somatic cell solution feeding channel 72 may be coated with poly-HEMA to render it non-cell-adherent, so that the somatic cells do not adhere. Alternatively, a material resistant to somatic cell adhesion may be used as the material for the second somatic cell solution feeding channel 72. Also, by using a material with good thermal diffusivity and CO₂ permeability as the material of the second somatic cell solution feeding channel 72, the conditions in the second somatic cell solution feeding channel 72 will be equivalent to the controlled temperature and CO₂ concentration in the enclosure 200. In addition, a back-flow valve may be provided in the second somatic cell solution feeding channel 72 from the viewpoint of preventing contamination.

The second somatic cell solution feeding channel 72 is connected to the second dissociating mechanism 80. The second dissociating mechanism 80 comprises a mesh, for example. The cell masses in the solution are dissociated into a plurality of cell masses of the sizes of the holes of the mesh, when they pass through the mesh by water pressure. If the mesh hole sizes are uniform, for example, the sizes of the plurality of cell masses after being dissociated will be approximately uniform. Alternatively, the second dissociating mechanism 80 may comprise a nozzle. For example, if the interior of an approximately conical nozzle is micromachined in a step-wise manner, a cell mass in the solution will be dissociated into a plurality of cell masses when it passes through the nozzle.

The somatic cell transport mechanism 90 that sends the somatic cells in order to the packaging device 100 is connected to the second dissociating mechanism 80 shown in Fig. 2. When cell masses do not form, the second dissociating mechanism 80 may be omitted. In this case, the second somatic cell solution feeding channel 72 is connected to the somatic cell transport mechanism 90.

A pre-packaging cell channel 91 is connected between the somatic cell transport mechanism 90 in the enclosure 200 and the packaging device 100. The somatic cell transport mechanism 90 employs a pump or the like to send the somatic cells to the packaging device 100 through the pre-packaging cell channel 91.

The pre-packaging cell channel 91 is coated with poly-HEMA so that the somatic cells do not adhere. Alternatively, a material resistant to somatic cell adhesion may be used as the material for the pre-packaging cell channel 91. Also, by using a material with good thermal diffusivity and CO₂ permeability as the material of the pre-packaging cell channel 91, the conditions in the pre-packaging cell channel 91 will be equivalent to the controlled temperature and CO₂ concentration in the enclosure 200. In addition, a back-flow valve may be provided in the pre-packaging cell channel 91 from the viewpoint of preventing contamination.

A cryopreservation liquid solution-feeding mechanism 110 is connected to the pre-packaging cell channel 91. The cryopreservation liquid solution-feeding mechanism 110 feeds a cell cryopreservation liquid into the pre-packaging cell channel 91. As a result, the somatic cells are suspended in the cell cryopreservation liquid inside the pre-packaging cell channel 91.

The packaging device 100 freezes the somatic cells in order, that have been fed through the pre-packaging cell channel 91. For example, each time it receives somatic cells, the packaging device 100 places the somatic cells in a cryopreservation vessel such as a cryotube, and immediately freezes the somatic cell-containing solution at -80°C or below, for example. When using a cryopreservation vessel with a small surface area per volume, more time will tend to be necessary for freezing, and therefore it is preferred to use a cryopreservation vessel with a large surface area per volume. By using a cryopreservation vessel with a large surface area per volume it is possible to increase the survival rate of the cells after thawing. The shape of the cryopreservation vessel may be capillary-like or spherical, without any particular restrictions. Immediate freezing is not necessarily essential, depending on the survival rate required for the cells after thawing.

Vitrification, for example, may be employed for the freezing. In this case, the cell cryopreservation liquid used may be DAP213 (Cosmo Bio Co., Ltd.) or Freezing Medium (ReproCELL, Inc.). The freezing may also be carried out by a common method other than vitrification. In this case, the cell cryopreservation liquid used may be CryoDefend-Stem Cell (R&D Systems) or STEM-CELLBANKER^{R} (Zenoaq). The freezing may be carried out with liquid nitrogen, or it may be carried out with a Peltier element. When a Peltier element is used, temperature changes can be controlled and temperature variation can be minimized. The packaging device 100 carries the cryopreservation vessel out of the enclosure 200. When the frozen cells are to be used in the clinic, the cryopreservation vessel is preferably a completely closed system. However, the packaging device 100 may package the somatic cells in a preservation vessel without freezing.

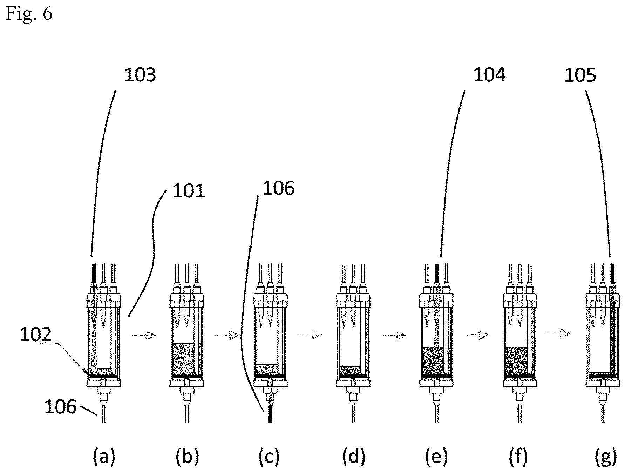

Alternatively, in the packaging device 100, the solvent of the somatic cell-containing solution may be exchanged from the culture medium to the cryopreservation liquid using a solution exchanger 101 as illustrated in Fig. 6. Inside the solution exchanger 101 there is provided a filter 102 having at the bottom a fine hole which does not permit passage of somatic cells. In the solution exchanger 101 there is also provided a somatic cell introduction hole where a first solution-feeding channel 103 that feeds somatic cell-containing culture medium onto the internal filter 102 is connected, an exchange solution introduction hole where a second solution-feeding channel 104 that feeds somatic cell-free frozen solution onto the internal filter 102 is connected, and a somatic cell outflow hole where a first discharge channel 105 that discharges somatic cell-containing frozen solution onto the internal filter 102 is connected. There is also provided in the solution exchanger 101 a waste liquid outflow hole wherein there is connected a second discharge channel 106 that discharges solution that has passed through the filter 102. Tubes or the like may be used for each of the first solution-feeding channel 103, second solution-feeding channel 104, first discharge channel 105 and second discharge channel 106.

First, as shown in Fig. 6(a) and Fig. 6(b), somatic cell-containing culture medium is placed inside the solution exchanger 101 from the first solution-feeding channel 103, while flow of the solution in the second discharge channel 106 is stopped. Next, as shown in Fig. 6(c), a state is formed allowing flow of the solution in the second discharge channel 106, whereby the culture medium is discharged from the solution exchanger 101. The somatic cells remain on the filter 102 during this time, as shown in Fig. 6(d). First, as shown in Fig. 6(e) and Fig. 6(f), the cryopreservation liquid is placed inside the solution exchanger 101 from the second solution-feeding channel 104, while flow of the solution in the second discharge channel 106 is stopped, and the somatic cells are dispersed in the cryopreservation liquid. Next, as shown in Fig. 6(g), the somatic cell-containing cryopreservation liquid is discharged from the first discharge channel 105. The somatic cell-containing cryopreservation liquid is sent to a cryopreservation vessel or the like through the first discharge channel 105.

The somatic cell production system of Fig. 1 and Fig. 2 may still further comprise a sterilizing device that performs sterilization inside the enclosure 200. The sterilizing device may be a dry heat sterilizing device. In this case, the wirings of the devices that use electricity, such as the separating device 10, preintroduction cell solution-feeding channel 20, inducing factor solution-feeding mechanism 21, factor introducing device 30, somatic cell preparation device 40 and packaging device 100, are preferably heat-resistant wirings. Alternatively, the sterilizing device may emit sterilizing gas such as ozone gas, hydrogen peroxide gas or formalin gas into the enclosure 200, to sterilize the interior of the enclosure 200.

The somatic cell production system may also record the behavior of the separating device 10, preintroduction cell solution-feeding channel 20, inducing factor solution-feeding mechanism 21, factor introducing device 30, somatic cell preparation device 40 and packaging device 100, and may transmit the image taken by the photographing device to an external server, in either a wired or wireless manner. In addition, the external server may control the separating device 10, inducing factor solution-feeding mechanism 21, factor introducing device 30, somatic cell preparation device 40 and packaging device 100 of the somatic cell production system based on a standard operation procedure (SOP), monitor whether or not each device is running based on the SOP, and automatically produce a running record for each device.

The somatic cell production system described above allows somatic cells to be automatically induced.

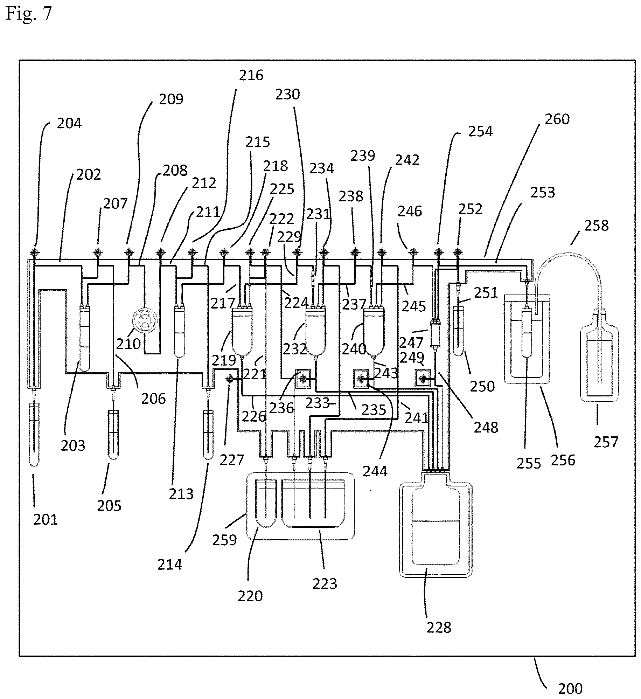

The somatic cell production system of this embodiment is not limited to the construction illustrated in Fig. 1 and Fig. 2. For example, in the somatic cell production system of the embodiment shown in Fig. 7, blood is delivered from the blood storing device 201 to the mononuclear cell separating unit 203, through a blood solution-feeding channel 202. Tubes, for example, may be used as the blood storing device 201 and mononuclear cell separating unit 203. The blood solution-feeding channel 202 may be a resin tube or silicon tube, for example. This also applies for the other solution-feeding channels described below. An identifier such as a barcode is attached to the blood storing device 201 for control of the blood information. A pump 204 is used for feeding of the solution. The pump 204 that is used may be a positive-displacement pump. Examples of positive-displacement pumps include reciprocating pumps including piston pumps, plunger pumps and diaphragm pumps, and rotating pumps including gear pumps, vane pumps and screw pumps. Examples of diaphragm pumps include tubing pumps and piezoelectric pumps. Examples of tubing pumps include Perista Pump^{R} (Atto Corp.) and RP-Q1 and RP-TX (Takasago Electric, Inc.). Examples of piezoelectric pumps include SDMP304, SDP306, SDM320 and APP-20KG (Takasago Electric, Inc.). A microflow chip module (Takasago Electric, Inc.) comprising a combination of various different pumps may also be used. When a sealed pump such as a Perista Pump^{R}, tubing pump or diaphragm pump is used, delivery can be accomplished without direct contact of the pump with the blood inside the blood solution-feeding channel 202. The same also applies to the other pumps described below. Alternatively, syringe pumps may be used for the pump 204, and for the pump 207, pump 216, pump 222, pump 225, pump 234, pump 242 and pump 252 described below. Even pumps other than sealed pumps may be reutilized after heat sterilization treatment.

An erythrocyte coagulant is fed to the mononuclear cell separating unit 203 from the separating agent storing device 205, through a solution-feeding channel 206 and the pump 207. Tubes, for example, may be used as the separating agent storing device 205. An identifier such as a barcode is attached to the separating agent storing device 205 for control of the separating agent information. The erythrocyte coagulant used may be, for example, HetaSep^{R} (STEMCELL Technologies) or an Erythrocyte Coagulant (Nipro Corp.). In the mononuclear cell separating unit 203, the erythrocytes precipitate by the erythrocyte coagulant and the mononuclear cells are separated. The mononuclear cell-containing supernatant in the mononuclear cell separating unit 203 is sent to a mononuclear cell purifying filter 210 through a mononuclear cell solution-feeding channel 208 and pump 209. At the mononuclear cell purifying filter 210, components other than the mononuclear cells are removed to obtain a mononuclear cell-containing solution as preintroduction cells. The mononuclear cell purifying filter 210 used may be Purecell^{R} (PALL), Cellsorba E (Asahi Kasei Corp.), SEPACELL PL (Asahi Kasei Corp.), ADACOLUMN^{R} (Jimro), or a separation bag (Nipro Corp.).

In Fig. 7, the mononuclear cell separating unit 203, separating agent storing device 205, mononuclear cell purifying filter 210 and pumps 204, 207, 209 constitute a separating device. When previously prepared preintroduction cells are to be used, however, the separating device may be omitted, as mentioned above.

The preintroduction cell-containing solution is sent to a factor introducing device 213 through a preintroduction cell solution-feeding channel 211 and pump 212. Tubes, for example, may be used as the factor introducing device 213. A somatic cell inducing factor is fed to the factor introducing device 213 from a factor storing device 214 that includes the somatic cell inducing factor, through the factor solution-feeding channel 215 and the pump 216. Tubes, for example, may also be used as the factor storing device 214. An identifier such as a barcode is attached to the factor storing device 214 for control of information relating to the somatic cell inducing factor. The factor storing device 214 and the pump 216 constitute the inducing factor solution-feeding mechanism. In the factor introducing device 213 serving as the factor introducing device, the somatic cell inducing factor is introduced into cells by RNA lipofection, for example, and inducing factor-introduced cells are prepared. The method of transfection of the inducing factor, however, is not limited to RNA lipofection. For example, Sendai virus vector including a somatic cell inducing factor may be used. Alternatively, the somatic cell inducing factor may be a protein. Transfection of the inducing factor may also be carried out several times over several days.

The inducing factor-introduced cells are sent through an introduced cell solution-feeding channel 217 and pump 218 to a somatic cell culturing vessel 219 as a part of the cell preparation device. The introduced cell solution-feeding channel 217 is, for example, temperature-permeable and CO₂-permeable. For the first few days after introduction of the somatic cell inducing factor to the cells, drug-containing cell culture medium is supplied to the somatic cell culturing vessel 219 from the cell medium storing device 220 including drug-containing cell culture medium, through the culture medium solution-feeding channel 221 and pump 222. The drug-containing cell culture medium includes a drug that kills cells into which the drug resistance factor has not been introduced. The culture medium solution-feeding channel 221 may be temperature-permeable and CO₂-permeable, for example. An identifier such as a barcode is attached to the drug-containing cell medium storing device 220 for control of the drug-containing cell medium information. The drug-containing cell medium storing device 220, culture medium solution-feeding channel 221 and pump 222 constitute the culture medium supply device.

Next, somatic cell culture medium is supplied to the somatic cell culturing vessel 219, from a somatic cell medium storing device 223 including somatic cell culture medium suited for the target somatic cells, through the culture medium solution-feeding channel 224 and pump 225. An identifier such as a barcode is attached to the somatic cell medium storing device 223 for control of the somatic cell culture medium information. The culture medium solution-feeding channel 224 may be temperature-permeable and CO₂-permeable, for example. The somatic cell medium storing device 223, culture medium solution-feeding channel 224 and pump 225 constitute the culture medium supply device.

The drug-containing cell medium storing device 220 and somatic cell medium storing device 223 may be placed in cold storage in the cold storage section 259 at a low temperature of 4°C, for example. The culture medium fed from the drug-containing cell medium storing device 220 and the somatic cell medium storing device 223 may be fed to the culturing vessel, for example, after having the temperature raised to 37°C with a heater outside the cold storage section 259. Alternatively, the temperature surrounding the solution-feeding channel may be set so that the culture medium stored at low temperature increases in temperature to 37°C while it progresses through the solution-feeding channel. The used culture medium in the somatic cell culturing vessel 219 is sent to a waste liquid storage section 228 through a waste liquid solution-feeding channel 226 and pump 227. An identifier such as a barcode is attached to the waste liquid storage section 228 for control of the waste liquid information.

The somatic cells that have been cultured with the somatic cell culturing vessel 219 are sent to a first amplifying culturing vessel 232 as a part of the cell preparation device, through the introduced cell solution-feeding channel 229, pump 230 and optionally the cell mass dissociater 231. By passing through the cell mass dissociater 231, the cell masses are dissociated into smaller cell masses. The cell mass dissociater 231 may be omitted if cell masses have not formed. Somatic cell culture medium is supplied to the first amplifying culturing vessel 232 from the somatic cell medium storing device 223 including the somatic cell culture medium, through the culture medium solution-feeding channel 233 and pump 234. The introduced cell solution-feeding channel 229 and culture medium solution-feeding channel 233 may be temperature-permeable and CO₂-permeable, for example. The somatic cell medium storing device 223, culture medium solution-feeding channel 233 and pump 234 constitute the culture medium supply device.

The used culture medium in the first amplifying culturing vessel 232 is sent to the waste liquid storage section 228 through a waste liquid solution-feeding channel 235 and pump 236.

The somatic cells that have been cultured at the first amplifying culturing vessel 232 are sent to a second amplifying culturing vessel 240 as a part of the cell preparation device, through an introduced cell solution-feeding channel 237, pump 238 and optionally the cell mass dissociater 239. By passing through the cell mass dissociater 239, the cell masses are dissociated into smaller cell masses. The cell mass dissociater 239 may be omitted if cell masses have not formed. Somatic cell culture medium is supplied to the second amplifying culturing vessel 240 from the somatic cell medium storing device 223 including the somatic cell culture medium, through the culture medium solution-feeding channel 241 and pump 242. The introduced cell solution-feeding channel 237 and culture medium solution-feeding channel 241 may be temperature-permeable and CO₂-permeable, for example. The somatic cell medium storing device 223, culture medium solution-feeding channel 241 and pump 242 constitute the culture medium supply device.

The used culture medium in the second amplifying culturing vessel 240 is sent to the waste liquid storage section 228 through a waste liquid solution-feeding channel 243 and pump 244.

The somatic cells that have been cultured in the second amplifying culturing vessel 240 are sent to a solution exchanger 247 through the introduced cell solution-feeding channel 245 and pump 246. The solution exchanger 247 comprises the construction shown in Fig. 6, for example. In the solution exchanger 247 shown in Fig. 7, the somatic cells are held at a filter while the culture medium is sent to the waste liquid storage section 228 through the waste liquid solution-feeding channel 248 and pump 249.

After stopping flow of the solution in the waste liquid solution-feeding channel 248 by stopping driving of the pump 249, or after closing the waste liquid solution-feeding channel 248 with a valve or the like, cryopreservation liquid is placed in the solution exchanger 247 from a cryopreservation liquid storing device 250, that contains cryopreservation liquid, through a solution-feeding channel 251 and pump 252. This disperses the somatic cells in the cryopreservation liquid.

The cryopreservation liquid that has dispersed the somatic cells is fed into a cryopreservation vessel 255 through a solution-feeding channel 253 and pump 254, as parts of the packaging device. The cryopreservation vessel 255 is situated in a low-temperature repository 256. Liquid nitrogen at -80°C, for example, is fed to the low-temperature repository 256 from a liquid nitrogen repository 257, through a solution-feeding channel 258. The somatic cells in the cryopreservation vessel 255 are thus frozen. However, freezing of the somatic cells does not need to be by liquid nitrogen. For example, the low-temperature repository 256 may be a freezer such as a compression freezer, an absorption freezer or a Peltier freezer. The somatic cells do not need to be frozen if freezing is not necessary.

Back-flow valves may also be provided in the solution-feeding channels as appropriate. The solution-feeding channels, mononuclear cell separating unit 203, mononuclear cell purifying filter 210, factor introducing device 213, somatic cell culturing vessel 219, first amplifying culturing vessel 232, second amplifying culturing vessel 240 and solution exchanger 247 are housed in a cassette-like case 260, for example, made of a resin or the like. The case 260 is made of a sterilizable heat-resistant material, for example. The case 260 is adjusted to an environment suitable for cell culture, such as 37°C, 5% CO₂ concentration. The solution-feeding channel through which the culture medium flows is made of a CO₂-permeable material, for example. However, the case 260 is not limited to a cassette-like form. It may instead be a flexible bag, for example. The solution-feeding channels, mononuclear cell separating unit 203, mononuclear cell purifying filter 210, factor introducing device 213, somatic cell culturing vessel 219, first amplifying culturing vessel 232, second amplifying culturing vessel 240 and solution exchanger 247 may also be housed in a plurality of separate cases.

The case 260 is disposed in the enclosure 200. The pump, blood storing unit 201, separating agent storing device 205, factor storing device 214, drug-containing cell medium storing device 220, somatic cell medium storing device 223, waste liquid storage section 228, cryopreservation vessel 255, low-temperature repository 256 and liquid nitrogen repository 257 are disposed inside the enclosure 200 and outside of the case 260.

The case 260 and enclosure 200 comprise engaging parts that mutually engage, for example. The case 260 will thus be disposed at a prescribed location in the enclosure 200. Furthermore, the pump, blood storing unit 201, separating agent storing device 205, factor storing device 214, drug-containing cell medium storing device 220, somatic cell medium storing device 223, waste liquid storage section 228, cryopreservation vessel 255, low-temperature repository 256 and liquid nitrogen repository 257 are also disposed at prescribed locations in the enclosure 200. When the case 260 is disposed at a prescribed location in the enclosure 200, the solution-feeding channels in the case 260 are in contact with the pump, blood storing unit 201, separating agent storing device 205, factor storing device 214, drug-containing cell medium storing device 220, somatic cell medium storing device 223, waste liquid storage section 228, cryopreservation vessel 255, low-temperature repository 256 and liquid nitrogen repository 257.

The case 260 and its contents may be disposable, for example, and upon completion of freezing of the somatic cells, they may be discarded and exchanged with new ones. Alternatively, when the case 260 and its contents are to be reused, an identifier such as a barcode may be attached to the case 260 to manage the number of times used, etc.

With the somatic cell production system of the embodiment described above, it is possible to automatically produce cryopreserved somatic cells such as iPS cells from preintroduction cells.

### (Other embodiments)

An embodiment of the invention has been described above, but the description and pertinent drawings that are intended merely to constitute part of the disclosure are not to be understood as limiting the invention. Various alternative embodiments, embodiments and operating technologies will be readily apparent to a person skilled in the art from this disclosure. For example, the factor introducing device 30 may induce the cells by a viral vector such as a retrovirus, lentivirus or Sendai virus, or by transfection using plasmids, or by protein transfection. Alternatively, the factor introducing device 30 may induce the cells by electroporation. Also, the preintroduction cell solution-feeding channel 20, introduced cell solution-feeding channel 31, first somatic cell solution feeding channel 51, amplifying culturing solution-feeding channel 71, second somatic cell solution feeding channel 72 and pre-packaging cell channel 91 may be provided on a substrate by a microfluidic technique. It will therefore be understood that the invention encompasses various embodiments not described herein.

### (Example 1)

A 12-well dish coated with a solubilized basal membrane preparation (Matrigel, Corning) was prepared, and feeder-free medium (mTeSR^{R} 1, Stemcell Technologies) containing ROCK (Rho-associated coiled-coil forming kinase/Rho bond kinase) inhibitor (Selleck) at a concentration of 10 µmol/L was added to each well. ROCK inhibitor inhibits cell death.

After dispersing iPS cells in a tissue and cultured cell detachment/separation/dispersion solution (Accutase, Innovative Cell Technologies), the dispersion was dispensed in a 12-well dish. The cells to be transfected were dispensed at a density of 4 × 10⁵ per well. The base area of each well was 4 cm². The non-transfected control cells were dispensed at a density of 2 × 10⁵ per well. The cells were then cultured in feeder-free medium for 24 hours. The temperature was 37°C, the CO₂ concentration was 5% and the oxygen concentration was ≤25%.

A transfection medium was prepared by mixing 1.25 mL of xeno-free medium (Pluriton, STEMGENT), 0.5 µL of Pluriton Supplement (STEMGENT) and 2 µL of 100 ng/µL B18R recombinant protein-containing solution (eBioscience). Before transfection, the feeder-free medium in each well was exchanged with transfection medium, and the cells were cultured at 37°C for 2 hours.

Green fluorescent protein (GFP) and mRNA (TriLink) were prepared. The mRNA was capped with Anti-Reverse Cap Analog (ARCA) and polyadenylated, and substituted with 5-methylcytidine and pseudouridine.

Also, a 1.5 mL micro centrifuge tube A and a 1.5 mL micro centrifuge tube B were prepared to match the number of wells.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture. Tube A was then lightly tapped for 10 minutes at room temperature, to mix the first reaction mixture.

In tube B there was placed 62.5 µL of low serum medium (Opti-MEM^{R}, Gibco), and then 500 ng of GFP mRNA (Trilink) was added and the mixture was thoroughly agitated to obtain a second reaction mixture.

The second reaction mixture was added to first reaction mixture in tube A to obtain a mixed reaction solution, and then tube A was lightly tapped for 5 minutes at room temperature to form liposomes. The mixed reaction solution was added to different wells and allowed to stand overnight at 37°C. Thus, 500 ng of GFP mRNA was added to each well.

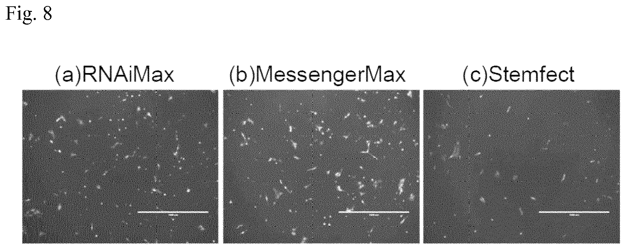

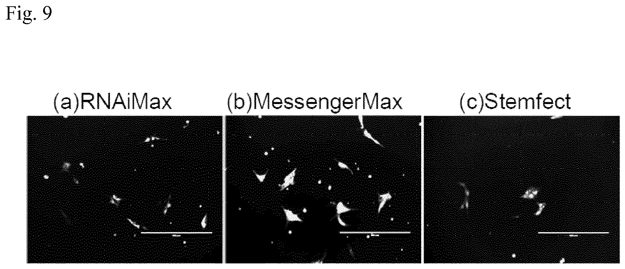

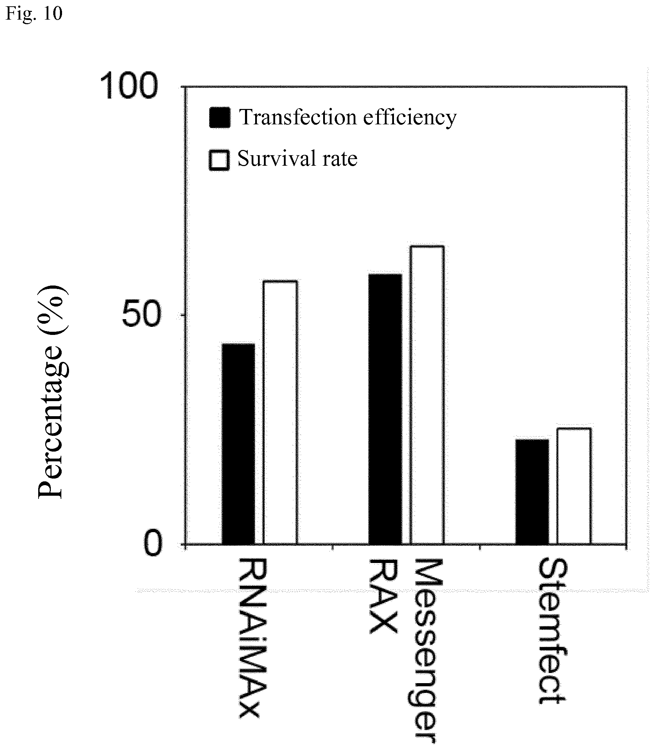

When a fluorescent microscope was used to obtain the cells on the following day, coloration of the transfected cells was confirmed, as shown in Fig. 8 and Fig. 9. The survival rate of the cells was also confirmed, as shown in Fig. 10. This indicated that expression of proteins was possible by introduction of mRNA into iPS cells using a lipofection reagent and RNA.

### (Example 2)

A 12-well dish coated with a solubilized basal membrane preparation (Matrigel, Corning) was prepared, and feeder-free medium (mTeSR^{R} 1, Stemcell Technologies) containing ROCK (Rho-associated coiled-coil forming kinase/Rho bond kinase) inhibitor (Selleck) at a concentration of 10 µmol/L was added to each well. ROCK inhibitor inhibits cell death.

After dispersing iPS cells in a tissue and cultured cell detachment/separation/dispersion solution (Accutase, Innovative Cell Technologies), the dispersion was dispensed in a 12-well dish. The cells to be transfected were dispensed at a density of 4 × 10⁵ per well. The non-transfected control cells were dispensed at a density of 2 × 10⁵ per well. The cells were then cultured in feeder-free medium for 24 hours.

A transfection medium was prepared by mixing 1.25 mL of xeno-free medium (Pluriton, STEMGENT), 0.5 µL of Pluriton Supplement (STEMGENT) and 2 µL of 100 ng/µL B18R recombinant protein-containing solution (eBioscience). Before transfection, the feeder-free medium in each well was exchanged with transfection medium, and the cells were cultured at 37°C for 2 hours.

Ngn2-T2A-Puro mRNA (Trilink), green fluorescent protein (GFP) and mRNA (Trilink) were prepared. The mRNA was capped with Anti-Reverse Cap Analog (ARCA) and polyadenylated, and substituted with 5-methylcytidine and pseudouridine. The mRNA was also purified with a silica membrane, and prepared as a solution in a solvent of 1 mmol/L sodium citrate at pH 6, together with mRNA-introducing reagent (Lipofectamine MessengerMax^{R}, Invitrogen). A 1.5 mL micro centrifuge tube A and a 1.5 mL micro centrifuge tube B were also prepared to match the number of wells.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture. Tube A was then lightly tapped for 10 minutes at room temperature, to mix the first reaction mixture.

In tube B there was placed 62.5 µL of low serum medium (Opti-MEM^{R}, Gibco), and then 500 ng of Ngn2-T2A-PuromRNA (Trilink) and 1500 ng of GFP mRNA (Trilink) were added and the mixture was thoroughly agitated to obtain a second reaction mixture.

The second reaction mixture was added to first reaction mixture in tube A to obtain a mixed reaction solution, and then tube A was lightly tapped for 5 minutes at room temperature to form liposomes. The mixed reaction solution was added to different wells and allowed to stand overnight at 37°C. Thus, 500 ng of Ngn2 mRNA and 100 ng of GFP mRNA were added to each well.

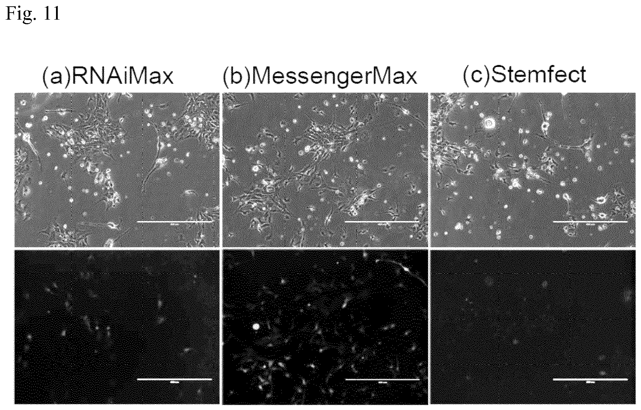

Coloration of the cells was confirmed on the first day after introduction of the mRNA, as shown in Fig. 11.

For 2 days thereafter, the medium was completely exchanged every day with nerve differentiation medium (N2/DMEM/F12/NEAA, Invitrogen) containing ROCK inhibitor (Selleck) at a concentration of 10 µmol/L and an antibiotic (puromycin) at a concentration of 1 mg/L, and the mRNA-transfected cells were selected. On the 3rd day, the medium was replaced with nerve differentiation medium (N2/DMEM/F12/NEAA, Invitrogen) containing a B18R recombinant protein-containing solution (eBioscience) at a concentration of 200 ng/mL. The medium was subsequently exchanged with the same medium in half the amount at a time, up until the 7th day.

On the 7th day, the medium was removed from the wells and rinsing was performed with 1 mL of PBS. After then adding 4% PFA, the mixture was reacted at 4°C for 15 minutes, and fixed. After then rinsing twice with PBS, primary antibody was diluted with 5% CCS, 0.1% Triton in PBS medium, and 500 µL was added. The primary antibodies used were rabbit anti-human Tuj1 antibody (BioLegend 845501) and mouse anti-rat and human Ngn2 antibody (R and D Systems), with the rabbit anti-human Tuj1 antibody (BioLegend 845501) diluted 1/1000 fold with buffer or the mouse anti-rat and human Ngn2 antibody (R and D Systems) diluted 1/75 fold with buffer, and DAPI diluted 1/10,000 fold with buffer was also added to each well, after which reaction was conducted for one hour at room temperature. Tuj1 antibody is antibody for β-III Tubulin.

After one hour of reaction at room temperature, 1 mL of PBS was added into each well and thoroughly mixed in the well, after which the PBS was discarded. PBS was again added and then discarded, and 500 µL of a secondary antibody-containing permeation buffer, which included 1/1000-fold diluted donkey anti-mouse IgG (H+L) secondary antibody Alexa Fluor^{R} 555 complex (Thermofisher) and 1/1000-fold diluted donkey anti-rabbit IgG (H+L) secondary antibody AlexaFluor^{R} 647 complex (Thermo fisher) in permeation buffer, was added to each well and reaction was conducted for 30 minutes at room temperature.

After reaction at room temperature for 30 minutes, the cells were rinsed twice with PBS and observed under a fluorescent microscope, and the fluorescence-emitting cells were counted.

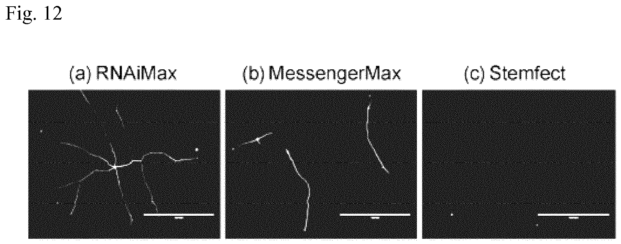

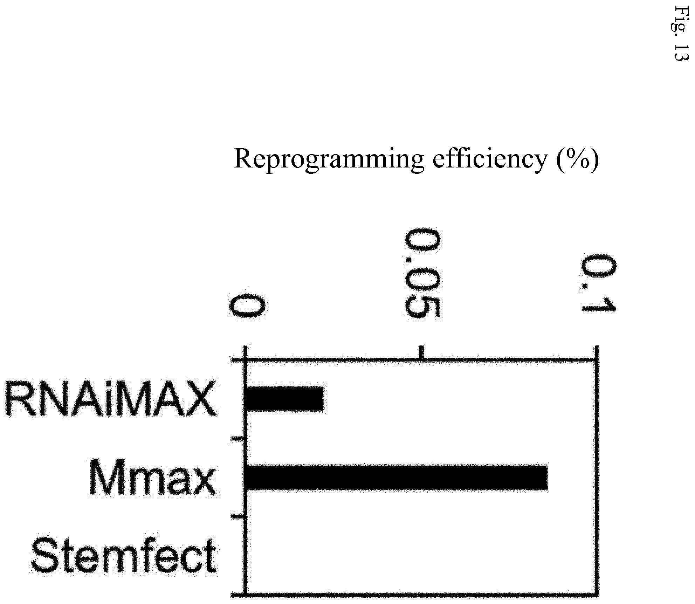

Fig. 12 is a photograph as observed with a fluorescent microscope after introducing Ngn2-T2A-Puro mRNA by lipofection and then adding puromycin and culturing for 2 days, and subsequently culturing for 5 days without adding puromycin, and staining with Tuji1. Fig. 13 shows the percentage of TUJ-1 positive cells on the 7th day after transfection of Ngn2-T2A-Puro mRNA using different transfection reagents by the procedure described above. The results show induction of neurons.

### (Example 3)

A 12-well dish coated with a solubilized basal membrane preparation (Matrigel, Corning) was prepared, and feeder-free medium (mTeSR^{R} 1, Stemcell Technologies) containing ROCK (Rho-associated coiled-coil forming kinase/Rho bond kinase) inhibitor (Selleck) at a concentration of 10 µmol/L was added to each well.

After dispersing iPS cells in a tissue and cultured cell detachment/separation/dispersion solution (Accutase, Innovative Cell Technologies), the dispersion was dispensed in a 12-well dish. The cells to be transfected were dispensed at a density of 4 × 10⁵ per well. The non-transfected control cells were dispensed at a density of 1 × 10⁵ per well. The cells were then cultured in feeder-free medium for 24 hours. The temperature was 37°C, the CO₂ concentration was 5% and the oxygen concentration was ≤25%.

A B18R-containing transfection medium was prepared by mixing 1.25 mL of xeno-free medium (Pluriton, STEMGENT), 0.5 µL of Pluriton Supplement (STEMGENT) and 2 µL of 100 ng/µL B18R recombinant protein-containing solution (eBioscience). A B18R-free transfection medium was also prepared by mixing 1.25 mL of xeno-free medium (Pluriton, STEMGENT) and 0.5 µL of Pluriton Supplement (STEMGENT).

Before transfection, the feeder-free medium in each well was exchanged with B18R-containing transfection medium or B18R-free transfection medium, and the cells were cultured at 37°C for 2 hours.

Ngn2-T2A-Puro mRNA (Trilink) and GFP mRNA (Trilink) were prepared. The mRNA was capped with Anti-Reverse Cap Analog (ARCA) and polyadenylated, and substituted with 5-methylcytidine and pseudouridine.

Also, a 1.5 mL micro centrifuge tube A and a 1.5 mL micro centrifuge tube B were prepared to match the number of wells.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture. Tube A was then lightly tapped for 10 minutes at room temperature, to mix the first reaction mixture.

In tube B there was placed 62.5 µL of low serum medium (Opti-MEM^{R}, Gibco), and then 500 ng of Ngn2-T2A-PuromRNA (Trilink) and 100 ng of GFP mRNA (Trilink) were added and the mixture was thoroughly agitated to obtain a second reaction mixture.

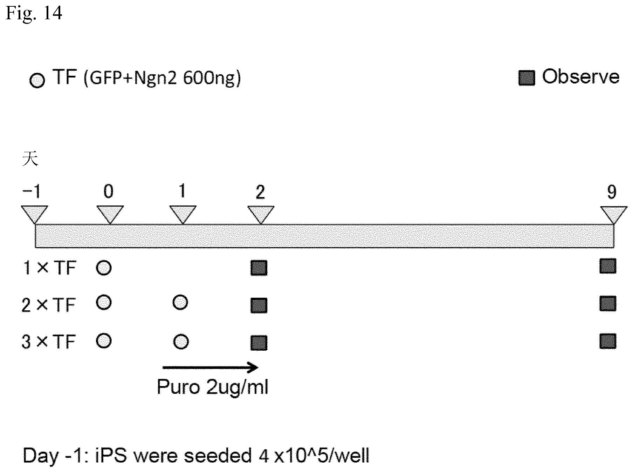

The second reaction mixture was added to first reaction mixture in tube A to obtain a mixed reaction solution, and then tube A was lightly tapped for 5 minutes at room temperature to form liposomes. The mixed reaction solution was added to different wells and allowed to stand overnight at 37°C. Thus, 500 ng of Ngn2 mRNA and 100 ng of GFP mRNA were added to each well. Cells that had been transfected 1, 2 and 3 times were prepared, as shown in Fig. 14.

For 2 days thereafter, the medium was completely exchanged every day with nerve differentiation medium (N2/DMEM/F12/NEAA, Invitrogen) containing ROCK inhibitor (Selleck) at a concentration of 10 µmol/L and an antibiotic (puromycin) at a concentration of 1 mg/L, and the mRNA-transfected cells were selected. On the 3rd day, the medium was replaced with nerve differentiation medium (N2/DMEM/F12/NEAA, Invitrogen) containing a B18R recombinant protein-containing solution (eBioscience) at a concentration of 200 ng/mL. The medium was subsequently exchanged with the same medium in half the amount at a time, up until the 7th day.

On the 7th day, the medium was removed from the wells and rinsing was performed with 1 mL of PBS. After then adding 4% PFA, the mixture was reacted at 4°C for 15 minutes, and fixed. After subsequently rinsing twice with PBS, primary antibody diluted with permeation buffer containing 5% CCS and 0.1% TritonX in PBS was added at 50 µL into each well, and reaction was conducted for 1 hour at room temperature. The primary antibody was diluted with permeation buffer so that the mouse anti-human Tuj1 antibody (BioLegend 845501) was at 1:1000 and the mouse anti-human Ngn2 antibody (R&D Systems, MAB3314-SP) was at 1:150, with addition of DAPI to 1:10,000.

After one hour, 1 mL of PBS was added into each well and thoroughly mixed in the well, and then the PBS was discarded. PBS was again added and then discarded, and 500 µL of a secondary antibody-containing permeation buffer, which included donkey anti-mouse IgG (H+L) secondary antibody Alexa Fluor^{R} 555 complex (Thermofisher, A-21428) at 1:1000 and donkey anti-rabbit IgG (H+L) secondary antibody AlexaFluor^{R} 647 complex (Thermofisher, A31573) at 1:1000 in permeation buffer, was added and reaction was conducted for 30 minutes at room temperature.

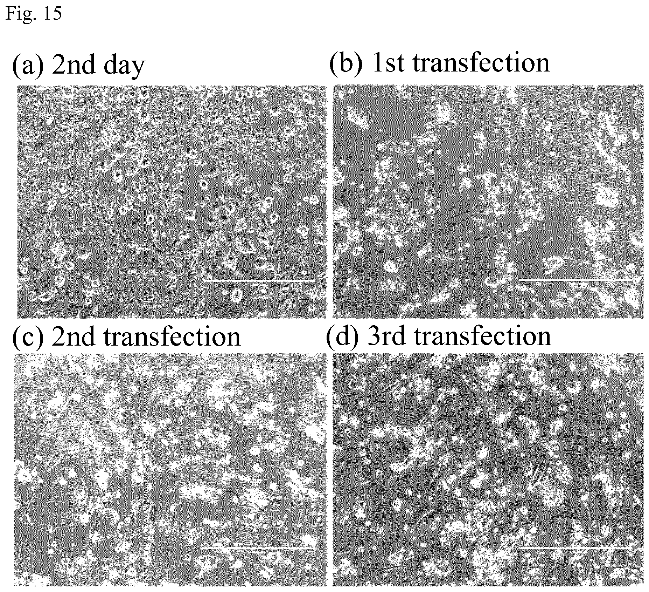

The cells were rinsed twice with PBS and observed under a fluorescent microscope, and the fluorescence-emitting cells were counted. As a result, as shown in Fig. 15, the cells transfected only once with mRNA exhibited virtually no GFP on the 9th day. On the other hand, the cells transfected 3 times with mRNA exhibited GFP even on the 9th day. This demonstrated that the mRNA is decomposed in the cells, and expression of the protein is transient.

As explained above, it was demonstrated that seeding of iPS cells followed by transfection of RNA can induce neurons within several days. Moreover, since induction of neurons is possible in a short period of time, this showed that the medium does not need to include B18R protein which is normally used to inhibit cell death caused by immunoreaction occurring during insertion of RNA into cells.

### Explanation of Symbols

2: Tube, 10: separating device, 20: preintroduction cell solution-feeding channel, 21: inducing factor solution-feeding mechanism, 30: factor introducing device, 31: introduced cell solution-feeding channel, 40: cell preparation device, 50: somatic cell culturing apparatus, 51: somatic cell solution-feeding channel, 60: dividing mechanism, 70: amplifying culturing apparatus, 71: amplifying culturing solution-feeding channel, 72: somatic cell solution-feeding channel, 80: dividing mechanism, 90: somatic cell transport mechanism, 91: pre-packaging cell channel, 100: packaging device, 101: solution exchanger, 102: filter, 103: feeding channel, 104: feeding channel, 105: discharge channel, 106: discharge channel, 110: cryopreservation liquid solution-feeding mechanism, 200: enclosure, 201: blood storing unit, 202: blood solution-feeding channel, 203: mononuclear cell separating unit, 204: pump, 205: separating agent storing device, 206: solution-feeding channel, 207: pump, 208: mononuclear cell solution-feeding channel, 209: pump, 210: mononuclear cell purifying filter, 211: preintroduction cell solution-feeding channel, 212: pump, 213: factor introducing device, 214: factor storing device, 215, 38: factor solution-feeding channel, 216: pump, 217: introduced cell solution-feeding channel, 218: pump, 219: somatic cell culturing vessel, 220: cell medium storing unit, 221: culture medium solution-feeding channel, 222: pump, 223: somatic cell medium storing device, 224: culture medium solution-feeding channel, 225: pump, 226: waste liquid solution-feeding channel, 227: pump, 228: waste liquid storage section, 229: introduced cell solution-feeding channel, 230: pump, 231: cell mass dissociater, 232: amplifying culturing vessel, 233: culture medium solution-feeding channel, 234: pump, 235: waste liquid solution-feeding channel, 236: pump, 237: introduced cell solution-feeding channel, 238: pump, 239: cell mass dissociater, 240: amplifying culturing vessel, 241: culture medium solution-feeding channel, 242: pump, 243: waste liquid solution-feeding channel, 244: pump, 245: introduced cell solution-feeding channel, 246: pump, 247: solution exchanger, 248: waste liquid solution-feeding channel, 249: pump, 250: cryopreservation liquid storing device, 251: solution-feeding channel, 252: pump, 253: solution-feeding channel, 254: pump, 255: cryopreservation vessel, 256: low-temperature repository, 257: liquid nitrogen repository, 258: solution-feeding channel, 259: cold storage section, 260: case, 301: bag, 302: bag

## Claims

1. A somatic cell production system comprising a preintroduction cell solution-feeding channel through which a preintroduction cell-containing solution passes, a factor introducing device that is connected to the preintroduction cell solution-feeding channel and introduces a somatic cell inducing factor into preintroduction cells to prepare inducing factor-introduced cells, and a cell preparation device in which the inducing factor-introduced cells are cultured to prepare somatic cells.

2. The somatic cell production system according to claim 1, which further comprises an enclosure that houses the preintroduction cell solution-feeding channel, factor introducing device and cell preparation device.

3. The somatic cell production system according to claim 1 or 2, wherein the somatic cells exclude pluripotent stem cells.

4. The somatic cell production system according to claim 1 or 2, wherein the somatic cells include differentiated cells.

5. The somatic cell production system according to claim 1 or 2, wherein the somatic cells include somatic stem cells.

6. The somatic cell production system according to claim 1 or 2, wherein the somatic cells include nervous system cells.

7. The somatic cell production system according to claim 1 or 2, wherein the preintroduction cells include pluripotent stem cells.

8. The somatic cell production system according to claim 1 or 2, wherein the preintroduction cells include somatic stem cells.

9. The somatic cell production system according to claim 1 or 2, wherein the preintroduction cells include differentiated somatic cells.

10. A somatic cell production system according to any one of claims 1 to 9, wherein the cell preparation device comprises a somatic cell culturing apparatus wherein the inducing factor-introduced cells created by the factor introducing device are cultured and an amplifying culturing apparatus wherein the somatic cells established by the somatic cell culturing apparatus are subjected to amplifying culturing, the somatic cell culturing apparatus comprises a first culture medium supply device that supplies culture medium to the inducing factor-introduced cells, and the amplifying culturing apparatus comprises a second culture medium supply device that supplies culture medium to the somatic cells.

11. The somatic cell production system according to claim 10, wherein the somatic cell culturing apparatus further comprises a drug supply device that feeds a solution containing a drug that kills cells in which a drug resistance factor has not been introduced.

12. The somatic cell production system according to any one of claims 1 to 11, wherein the factor introducing device comprises a factor introducing device connected to the preintroduction cell solution-feeding channel, a factor storing device that stores the somatic cell inducing factor, a factor solution-feeding channel for streaming of the somatic cell inducing factor from the factor storing device to the preintroduction cell solution-feeding channel or factor introducing device, and a pump for streaming of the liquid in the factor solution-feeding channel.

13. The somatic cell production system according to claim 12, wherein the somatic cell inducing factor is DNA, RNA or protein.

14. The somatic cell production system according to claim 12, wherein the somatic cell inducing factor is introduced into the preintroduction cells by RNA lipofection at the factor introducing device.

15. The somatic cell production system according to claim 12, wherein the somatic cell inducing factor is incorporated into a vector.

16. The somatic cell production system according to claim 15, wherein the vector is Sendai virus vector.

17. The somatic cell production system according to any one of claims 1 to 16, which further comprises a packaging device that packages the somatic cells created by the cell preparation device, and the enclosure houses the packaging device.

18. The somatic cell production system according to any one of claims 1 to 17, which further comprises a solution exchanger comprising a tubular member and a liquid permeable filter disposed inside the tubular member, the solution exchanger being provided with, in the tubular member, a somatic cell introduction hole for introduction of a solution including somatic cells created by the cell preparation device, onto the liquid permeable filter, an exchange solution introduction hole for introduction of exchange solution onto the liquid permeable filter, a somatic cell outflow hole for outflow of the exchange solution including the somatic cells onto the liquid permeable filter, and a waste liquid outflow hole through which the solution that has permeated the liquid permeable filter flows out.

19. The somatic cell production system according to claim 18, which further comprises a waste liquid solution-feeding channel connected to the waste liquid outflow hole, permitting the solution containing the somatic cells to flow through the waste liquid solution-feeding channel when the solution is discarded, or not permitting the solution to flow through the waste liquid solution-feeding channel when the somatic cells are being dispersed in the exchange solution.

20. The somatic cell production system according to claim 18 or 19, wherein the exchange solution is a cryopreservation liquid.

21. The somatic cell production system according to claim 1, which further comprises a separating device that separates preintroduction cells from blood, wherein the preintroduction cell-containing solution separated by the separating device passes through the preintroduction cell solution-feeding channel.
